# EUROPEAN PATENT SPECIFICATION

(11) **EP 1 756 265 B1**
(45) Date of publication and mention of the grant of the patent: **22.01.2014**
(21) Application number: 05736004.2
(22) Date of filing: 22.04.2005
(51) Int. Cl.: C12N 1/20, C12P 19/04

(54) **SOY PEPTONE AS A NITROGEN SOURCE IN PREPARING MENINGOCOCCAL CONJUGATES**
SOJAPEPTON ALS STICKSTOFFQUELLE BEI DER HERSTELLUNG VON MENINGOKOKKENKONJUGATEN
PEPTONE DE SOJA SERVANT DE SOURCE D'AZOTE POUR PREPARER DES CONJUGUES MENINGOCOCCIQUES

(30) Priority: 22.04.2004 GB 0408978
(43) Date of publication of application: 28.02.2007
(73) Proprietor: Novartis AG, 4056 Basel (CH)
(72) Inventor: MARSHALL, Cameron John, London WC1A 2RA (GB)
(74) Representative: Marshall, Cameron John
(86) International application number: PCT/GB2005/001543
(87) International publication number: WO 2005/103230

(56) References cited:
- EP-A- 0 528 635
- WO-A-98/54296
- WO-A-02/058737
- WO-A-03/007985
- WO-A-2004/033623
- US-A- 4 351 761
- US-A- 4 814 276
- US-A- 5 494 808
- MORIE G ET AL: "Development of a fermentation medium free from animal derived components for the production of polysaccharides from Neisseria meningitidis Group A and Group C" ABSTRACTS OF THE GENERAL MEETING OF THE AMERICAN SOCIETY FOR MICROBIOLOGY, vol. 97, 4 May 1997 (1997-05-04), page 183, XP008055404 ISSN: 1060-2011
- FU J ET AL: "Recent advances in the large scale fermentation of Neisseria meningitidis group B for the production of an outer membrane protein complex." BIO/TECHNOLOGY, vol. 13, no. 2, February 1995 (1995-02), pages 170-174, XP001009628 ISSN: 0733-222X
- PELTOLA H: "MENINGOCOCCAL VACCINES CURRENT STATUS AND FUTURE POSSIBILITIES" DRUGS, ADIS INTERNATIONAL LTD, AT, vol. 55, no. 3, March 1998 (1998-03), pages 347-366, XP008022620 ISSN: 0012-6667

## Description

### TECHNICAL FIELD

This invention concerns vaccines against *Neisseria meningitidis.* In particular, it concerns vaccines based on conjugated capsular saccharides from meningococcus.

### BACKGROUND ART

*Neisseria meningitidis* (meningococcus) is a Gram negative human pathogen. It colonises the pharynx, causing meningitis and, occasionally, septicaemia in the absence of meningitis. It is closely related to *N.gonorrhoeae,* although one feature that clearly differentiates meningococcus is the presence of a polysaccharide capsule that is present in all pathogenic meningococci.

Based on the organism's capsular polysaccharide, twelve serogroups of *N.meningitidis* have been identified (A, B, C, H, I, K, L, 29E, W135, X, Y and Z). Group A is the pathogen most often implicated in epidemic disease in sub-Saharan Africa. Serogroups B and C are responsible for the vast majority of cases in USA and in most developed countries. Serogroups W135 and Y are responsible for the remaining cases in USA and developed countries.

A tetravalent vaccine of capsular polysaccharides from serogroups A, C, Y and W135 has been known for many years [1, 2] and has been licensed for human use. Although effective in adolescents and adults, it induces a poor immune response and short duration of protection and cannot be used in infants [*e.g.* 3] . This is because polysaccharides are T cell-independent antigens that induce a weak immune response that cannot be boosted. The polysaccharides in this vaccine are not conjugated and are present at a 1:1:1:1 ratio [4]. MENCEVAX ACWY™ contains 50µg of each purified polysaccharide once reconstituted from its lyophilised form.

Conjugate vaccines against serogroup C have been approved for human use, and include Menjugate™ [5], Meningitec™ and NeisVac-C™. Mixtures of conjugates from serogroups A+C are known [6,7] and from serogroups A+C+W135+Y have been reported [8-11].

In reference 8, meningococci are prepared initially using liquid Watson Scherp medium, then using Mueller Hinton agar medium, then using Watson Scherp medium again. The fermenter vessel was incubated at 35-37°C, controlling dissolved oxygen content and pH with supplement feed and antifoam additions. In reference 9, meningococci were grown in Franz A as medium, followed by further growth in Watson's medium. In both cases, bacteria were then harvested, then polysaccharides extracted, and then they were covalently linked to carrier proteins to give conjugate vaccines.

It is an object of the invention to provide further and improved media and methods for growing meningococci for the preparation of conjugate vaccines.

### DISCLOSURE OF THE INVENTION

Reference 12 discloses animal-free fermentation media for growing meningococci in all of serogroups A, C, W135 and Y, with the aim being to prepare capsular polysaccharides. According to the present invention, these media are used as described in reference 12, and the purified polysaccharides are then used for preparing conjugated vaccines for protecting against meningococci.

The invention is defined in the claims. According to claim 1, in a first aspect, the invention provides a method for preparing a protein-saccharide conjugate, comprising the steps of:
(a) preparing an aqueous growth medium comprising: (i) 2.5 g/l sodium phosphate, dibasic; (ii) between 5 and 30 g/l soy peptone; (iii) 5 g/l monosodium glutamate; (iv) 0.103 g/l potassium chloride; (v) 0.732 g/l magnesium sulfate; (vi) 11.250 g/l glucose; and, optionally, (vii) 0.016 g/l L-cysteine; but not including ammonium chloride;
(b) inoculating the medium with a *Neisseria meningitidis* bacterium;
(c) incubating the medium to allow growth of the bacterium;
(d) preparing capsular saccharide from the bacterium; and
(e) conjugating the capsular saccharide to a carrier protein, to give the a protein-saccharide conjugate,
wherein the concentrations of components of the medium may vary by ± 10%.

In a second aspect, the invention provides a method for preparing a protein-saccharide conjugate, comprising the steps of:
(a) preparing an aqueous growth medium comprising: (i) 10 g/l glucose; (ii) between 5 and 30 g/l soy peptone; (iii) 5.80 g/l sodium chloride; (iv) 1 g/l potassium sulfate; (v) 4 g/l potassium phosphate, dibasic; (vi) 0.19 g/l magnesium chloride; (vii) 0.021 g/l calcium chloride; (viii) 0.002 g/l ferrous sulfate; and, optionally, a mixture of amino acids comprising 5 g/l L-glutamic acid, 0.3 g/l L-arginine, 0.5 g/l L-serine and 0.23 g/l L-cysteine, but not including ammonium chloride;
(b) inoculating the medium with a *Neisseria meningitidis* bacterium;
(c) incubating the medium to allow growth of the bacterium;
(d) preparing capsular saccharide from the bacterium; and
(e) conjugating the capsular saccharide to a carrier protein, to give the a protein-saccharide conjugate,
wherein the concentrations of components of the medium may vary by ± 10%.

Further aspects of the invention are defined in claims 2 - 23.

Described herein are methods for preparing a protein-saccharide conjugate, comprising the steps of:
(a) growing *Neisseria meningitidis* in an aqueous medium comprising soy peptone as a nitrogen source;
(b) preparing capsular saccharide from the bacterium; and
(c) conjugating the capsular saccharide to a carrier protein, to give the a protein-saccharide conjugate.

Described herein are methods for preparing a protein-saccharide conjugate, comprising the steps of:
(a) preparing an aqueous growth medium comprising soy peptone as a nitrogen source;
(b) inoculating the medium with a *Neisseria meningitidis* bacterium;
(c) incubating the medium to allow growth of the bacterium;
(d) preparing capsular saccharide from the bacterium; and
(e) conjugating the capsular saccharide to a carrier protein, to give the a protein-saccharide conjugate.

The disclosure herein relates to soy peptone for use as the nitrogen source during growth of *N.meningitidis* for preparing capsular saccharide for conjugation to a protein carrier.

### The aqueous medium

A first aqueous medium used in the methods of the invention comprises: (i) 2.5 g/l sodium phosphate, dibasic; (ii) between 5 and 30 g/l soy peptone; (iii) 5 g/l monosodium glutamate;(iv) 0.103 g/l potassium chloride; (v) 0.732 g/l magnesium sulfate; and (vi) 11.250 g/l glucose (D- and/or L-glucose). As described in reference 12, the medium may also include 0.016 g/l L-cysteine. Also described herein is, more generally, a medium comprising (i) between 1 and 5 g/l sodium phosphate, dibasic; (ii) between 1 and 50 g/l soy peptone; (iii) between 2 and 10 g/l monosodium glutamate;
(iv) between 20 and 200 mg/l potassium chloride; (v) between 500 and 1000 mg/l magnesium sulfate; and (vi) between 5 and 20 g/l glucose (D- and/or L-glucose). The medium may include between 5 and 30 mg/l L-cysteine. Optimum concentrations within these ranges (e.g. to maximise polysaccharide yield, particularly for serogroup A) can readily be determined by routine experiments.

A second aqueous medium used in the methods of the invention comprises: (i) 10 g/l glucose; (ii) between 5 and 30 g/l soy peptone; (iii) 5.8 g/l sodium chloride; (iv) 1 g/l potassium sulfate; (v) 4 g/l potassium phosphate, dibasic; (vi) 0.19 g/l magnesium chloride; (vii) 0.021 g/l calcium chloride; (viii) 0.002 g/l ferrous sulfate. As described in reference 12, the medium may also include L-amino acids, such that the medium may comprise: between 5 and 6 g/l L-glutamic acid, 0.3 g/l L-arginine, 0.5 g/l L-serine and/or 0.23 g/l L-cysteine.

Also disclosed herein is, more generally, a medium comprising (i) between 2 and 20 g/l glucose; (ii) between 1 and 50 g/l soy peptone; (iii) between 2 and 10 g/l sodium chloride; (iv) between 0.2 and 4 g/l potassium sulfate; (v) between 1 and 10 g/l potassium phosphate, dibasic; (vi) between 50 and 400 mg/l magnesium chloride; (vii) between 5 and 50 mg/l calcium chloride; (viii) between 0.5 and 5 mg/l ferrous sulfate. The medium may also include L-amino acids, such that the medium may comprise: between 1 and 10 g/l L-glutamic acid, between 0.1 and 5 g/l L-arginine, between 0.1 and 5 g/l L-serine and/or between 0.05 and 0.5 g/l L-cysteine. Optimum concentrations within these ranges (e.g. to maximise polysaccharide yield, particularly for serogroup A) can readily be determined by routine experiments.

These media can be preapred by simply dissolving the indicated components in water e.g. in pure water, such as distilled water. The water is preferably sterile.

The medium may contain other components (e.g. further components that do not inhibit meningococcal growth, such as foam control agent e.g. Dow 1520 antifoam solution), but it does not include ammonium chloride (NH₄Cl) *i.e.* no ammonium chloride is used during preparation of the medium, and the concentration of ammonium chloride in the medium is less than 1 mg/ml. Ammonium chloride has been found to be not readily consumed during *Neisseria* fermentation, and it may even be deleterious to growth.

Rather than use ammonium chloride in the media, another nitrogen source is used, namely soy peptone, and a preferred soy peptone is HSP-A®. Thus a preferred soy peptone has the following characteristics: light tan colour; 51 % protein; 8% total nitrogen, 3% amino nitrogen; an AN/TN ration of 0.38; <10% ash; <8% moisture; pH 6.5; 1% sodium; and 4% potassium. The amino acid profile of a preferred soy peptone is given in table 1 below. Other soy peptones include SE50MAF-UF, Freetone A-1 and HY Soy UF. There are various other commercially-available soy peptones. Soy peptone may be used at between 5 g/l and 30 g/l, preferably between 10 g/l and 15 g/l. The concentrations of components of the medium are expressed in grams per liter (g/l), and may vary e.g. by ± 10%.

The pH of the medium, after aqueous reconstitution, is preferably 6.8±0.2.

**Table 1: Amino acid profile (mg/mg) of a preferred soy peptone:**

| **Amino acid** | **Free** | **Total** | | **Amino acid** | **Free** | **Total** |
|---|---|---|---|---|---|---|
| Asp | 6 | 45 | | Cys | N/A | 5 |
| Ser | 9 | 30 | | Tyr | 5 | 15 |
| Glu | 15 | 85 | | Val | 8 | 20 |
| Gly | 2 | 20 | | Met | 4 | 5 |
| His | 6 | 15 | | Lys | 16 | 30 |
| Arg | 14 | 40 | | Ile | 9 | 20 |
| Thr | 5 | 20 | | Leu | 19 | 30 |
| Ala | 5 | 20 | | Phe | 11 | 20 |
| Pro | 3 | 25 | | *TOTAL* | *137* | *445* |

The media defined above may be used to grow serogroup B of *N.meningitidis,* or to grow *N.gonorrhoeae.*

### Bacterial inoculatoin, incubation and growth

After media are prepared as described above, meningococci are introduced and the medium is then incubated to allow bacterial growth.

The method of the invention may involve multiple sub-steps of growth within the single "growth" step. Thus bacteria can be grown in a first medium, transferred into a second medium for further growth, transferred into a third medium, *etc.* Serial sub-steps may increase in volume *e.g.* volume may progress from a 1 litre flask to a 2.8 litre flask and then to 400 litre flask *etc.*

Preferred cultures take place at 30-40°C *e.g.* 36±1°C. Preferred cultures take place with a dissolved O₂ concentration of 30%. Preferred cultures take place with an airflow of 15 L/min. Preferred cultures take place with shaking *e.g.* at 250rpm. Preferred cultures are monitored for pH during growth, with pH being controlled by adding 2.5M phosphoric acid and/or 2.5M sodium hydroxide. A preferred bacterial growth involves fed-batch culture. A preferred feed solution for use during the culture comprises: 50 g/l glucose; 50 g/l glutamic acid; 3 g/l arginine; 3 g/l serine; 2 g/l cysteine; 10 g/l NH₄Cl; 2 g/l MgCl₂; 0.14 g/l CaCl₂; and 0.02 g/l FeSO₄.

Thus the invention provides a feed solution for use during meningococcal culture comprises: 50 g/l glucose; 50 g/l L-glutamic acid; 3 g/l L-arginine; 3 g/l L-serine; 2 g/l L-cysteine; 10 g/l NH₄Cl; 2 g/l MgCl₂; 0.14 g/l CaCl₂; and 0.02 g/l FeSO₄. The glucose can comprise D-glucose and/or L-glucose. The solution may be in its aqueous form, or may be in a dried form for reconstitutiing into the aqueous form. As before, the concentrations of components of this feed solution are expressed in grams per liter (g/l), and may vary *e.g.* by ± 10%.

### The bacteria

The invention involves the growth of *Neisseria meningitidis* bacteria. These may be of serogroups A, C, W135 or Y. A single culture preferably includes a single bacterial strain. The phrase "inoculating the medium with a *Neisseria meningitidis* bacterium" does not mean that only a single bacterium cell is introduced, but means that a single type of bacterium is introduced.

The capsular saccharides of the bacteria may be O-acetylated (OAc⁺ bacteria) or may not be O-acetylated (OAc⁻ bacteria). Each serogroup may be OAc⁺ or OAc⁻. Thus the invention may involve growth of an OAc⁺ serogroup C strain or an OAc⁻ serogroup C strain. It may involve growth of an OAc⁺ serogroup A strain or an OAc⁻ serogroup A strain. It may involve growth of an OAc⁺ serogroup W135 strain or an OAc⁻ serogroup W135 strain. It may involve growth of an OAc⁺ serogroup Y strain or an OAc⁻ serogroup Y strain.

### Polysaccharide preparation

Methods for preparing capsular saccharides from meningococcus are well known in the art e.g. see references 8, 9, 13, 14, 15, 16 *etc.*

One preferred method for preparing the saccharides involves polysaccharide precipitation followed by solubilisation of the precipitated polysaccharide using a lower alcohol [9]. Precipitation can be achieved using a cationic detergent such as tetrabutylammonium and cetyltrimethylammonium salts (*e.g.* the bromide salts), or hexadimethrine bromide and myristyltrimethylammonium salts. Cetyltrimethylammonium bromide ('CTAB') is particularly preferred [17]. Solubilisation of the precipitated material can be achieved using a lower alcohol such as methanol, propan-1-ol, propan-2-ol, butan-1-ol, butan-2-ol, 2-methyl-propan-1-ol, 2-methyl-propan-2-ol, diols, *etc.,* but ethanol is particularly suitable for solubilising CTAB-polysaccharide complexes. Ethanol is preferably added to the precipitated polysaccharide to give a final ethanol concentration (based on total content of ethanol and water) of between 50% and 95%. After re-solubilisation, the polysaccharide may be further treated to remove contaminants. This is particularly important in situations where even minor contamination is not acceptable (*e.g.* for human vaccine production). This will typically involve one or more steps of filtration *e.g.* depth filtration, filtration through activated carbon may be used, size filtration and/or ultrafiltration. Once filtered to remove contaminants, the polysaccharide may be precipitated for further treatment and/or processing. This can be conveniently achieved by exchanging cations (*e.g.* by the addition of calcium or sodium salts).

Another preferred method [8] for preparing the saccharides involves CTAB addition, centrifugation, and collection of supernatant. It may comprise a further round of precipitation with CTAB, centrifugation, and supernatant colleciton, to provide a paste. The paste may be blended with calcium chloride to give a homogeneous suspension. The suspension can be centrifuged, and the supernatant can be collected *e.g.* by decanting. Saccharide may be treated by ultrifiltration. Magnesium chloride can then be added, pH can be adjusted to 7.2 to 7.5 (*e.g.* using sodium hydroxide), and nucleases added. Ethanol can then be added to precipirate nucleic acid and protein. Precipitated material can eb removed by centrifugation. Saccharides in the supernatant can be recovered and precipitated by adding ethanol. The saccharide can then be dried, and then dissolved into sodium acetate solution.

The polysaccharide is preferably finally prepared as a dried powder, ready for conjugation.

### Conjugate preparation

After culture of bacteria and preparation of capsular polysaccharides, the saccharides are conjugated to carrier protein(s). In general, conjugation enhances the immunogenicity of saccharides as it converts them from T-independent antigens to T-dependent antigens, thus allowing priming for immunological memory. Conjugation is particularly useful for paediatric vaccines [*e.g.* ref. 18] and is a well known technique [*e.g.* reviewed in refs. 19 to 27].

Preferred carrier proteins are bacterial toxins or toxoids, such as diphtheria toxoid or tetanus toxoid. The CRM₁₉₇ mutant of diphtheria toxin [28-30] is a particularly preferred carrier for meningoccal conjugates, as is a diphtheria toxoid. Other suitable carrier proteins include the *N.meningitidis* outer membrane protein [31], synthetic peptides [32,33], heat shock proteins [34,35], pertussis proteins [36, 37], cytokines [38], lymphokines [38], hormones [38], growth factors [38], artificial proteins comprising multiple human CD4⁺ T cell epitopes from various pathogen-derived antigens [39], protein D from *H.influenzae* [40,41], pneumococcal surface protein PspA [42], iron-uptake proteins [43], toxin A or B from *C.difficile* [44], *etc.*

It is possible to use more than one carrier protein e.g. to reduce the risk of carrier suppression. Thus different carrier proteins can be used for different serogroups e.g. serogroup A saccharides might be conjugated to CRM₁₉₇ while serogroup C saccharides might be conjugated to tetanus toxoid. It is also possible to use more than one carrier protein for a particular saccharide antigen *e.g.* serogroup A saccharides might be in two groups, with some conjugated to CRM₁₉₇ and others conjugated to tetanus toxoid. In general, however, it is preferred to use the same carrier protein for all saccharides.

A single carrier protein might carry more than one saccharide antigen [45]. For example, a single carrier protein might have conjugated to it saccharides from serogroups A and C. To achieve this goal, saccharides can be mixed prior to the conjugation reaction. In general, however, it is preferred to have separate conjugates for each serogroup.

Conjugates with a saccharide:protein ratio (w/w) of between 1:5 (*i.e.* excess protein) and 5:1 (*i.e.* excess saccharide) are preferred. Ratios between 1:2 and 5:1 are preferred, as are ratios between 1:1.25 and 1:2.5 are more preferred. Excess carrier protein may be preferred for MenA and MenC.

Conjugates may be used in conjunction with free carrier protein [46]. When a given carrier protein is present in both free and conjugated form in a composition of the invention, the unconjugated form is preferably no more than 5% of the total amount of the carrier protein in the composition as a whole, and more preferably present at less than 2% by weight.

Any suitable conjugation reaction can be used, with any suitable linker where necessary.

The saccharide will typically be activated or functionalised prior to conjugation. Activation may involve, for example, cyanylating reagents such as CDAP (*e.g.* 1-cyano-4-dimethylamino pyridinium tetrafluoroborate [47,48,*etc*.]). Other suitable techniques use carbodiimides, hydrazides, active esters, norborane, p-nitrobenzoic acid, N-hydroxysuccinimide, S-NHS, EDC, TSTU; see also the introduction to reference 25).

Linkages via a linker group may be made using any known procedure, for example, the procedures described in references 49 and 50. One type of linkage involves reductive amination of the polysaccharide, coupling the resulting amino group with one end of an adipic acid linker group, and then coupling a protein to the other end of the adipic acid linker group [23, 51, 52]. Other linkers include B-propionamido [53], nitrophenyl-ethylamine [54], haloacyl halides [55], glycosidic linkages [56], 6-aminocaproic acid [57], ADH [58], C₄ to C₁₂ moieties [59] *etc.* As an alternative to using a linker, direct linkage can be used. Direct linkages to the protein may comprise oxidation of the polysaccharide followed by reductive amination with the protein, as described in, for example, references 60 and 61.

A process involving the introduction of amino groups into the saccharide (e.g. by replacing terminal =O groups with -NH₂) followed by derivatisation with an adipic diester (e.g. adipic acid N-hydroxysuccinimido diester) and reaction with carrier protein is preferred. Another preferred reaction uses CDAP activation with a protein D carrier e.g. for MenA or MenC.

After conjugation, free and conjugated saccharides can be separated. There are many suitable methods, including hydrophobic chromatography, tangential ultrafiltration, diafiltration *etc*. [see also refs. 62 & 63, *etc.*]*.*

Where the composition of the invention includes a depolymerised oligosaccharide, it is preferred that depolymerisation precedes conjugation *e.g.* is before activation of the saccharide.

In one preferred conjugation method, a saccharide is reacted with adipic acid dihydrazide. For serogroup A, carbodiimide may also be added at this stage. After a reaction period, sodium cyanoborohydride is added. Derivatised saccharide can then be prepared *e.g.* by ultrafiltration. The derivatized saccharide is then mixed with carrier protein (*e.g.* with a diphtheria toxoid), and carbodiimide is added. After a reaction period, the conjugate can be recovered. Further details of this conjugation method can be found in reference 8.

### Other steps

As well as including the steps described above, methods of the invention may include further steps. For example, the methods may include a step of depolymerisation of the capsular saccharides, after they are prepared from the bacteria but before conjugation. Depolymerisation reduces the chain length of the saccharides.

A preferred depolymerisation method involves the use of hydrogen peroxide [8]. Hydrogen peroxide is added to a saccharide (*e.g.* to give a final H₂O₂ concentration of 1%), and the mixture is then incubated (*e.g.* at around 55°C) until a desired chain length reduction has been achieved. The reduction over time can be followed by removing samples from the mixture and then measuring the (average) molecular size of saccharide in the sample. Depolymerization can then be stopped by rapid cooling once a desired chain length has been reached.

After conjugation, the methods of the invention may include a step of measuring the level of unconjugated carrier protein. One way of making this measurement involves capillary electrophoresis [64] (*e.g.* in free solution), or micellar electrokinetic chromatography [65].

After conjugation, the methods of the invention may include a step of measuring the level of unconjugated saccharide. One way of making this measurement involves HPAEC-PAD [62].

After conjugation, the methods of the invention may include a step of separating conjugated saccharide from unconjugated saccharide. One way of separating these saccharides is to use a method that selectively precipitates one component. Selective precpitation of conjugated saccharide is preferred, to leave unconjugated saccharide in solution, *e.g.* by a deoxycholate treatment [62].

After conjugation, the methods of the invention may include a step of measuring the molecular size and/or molar mass of a conjugate. In particular, distributions may be measured.. One way of making these measurements involves size exclusion chromatography with detection by multiangle light scattering photometry and differential refractometry (SEC-MALS/RI) [66].

### Conjugate combinations

Individual conjugates can be prepared as described above, for each of serogroups A, C, W135 and Y. The individual conjugates can then be mixed, in order to provide a tetravalent mixture.

It is also possible to mix fewer than four conjugates (*e.g.* to mix A+C, A+W135, A+Y, A+C+W135, A+C+Y, or A+W135+Y) to provide a bivalent or trivalent mixture.

Conjugates may be mixed by adding them individuall to a solution of phosphate buffered physiological saline (final concentration 10mM sodium phosphate). A preferred concentration of each conjugate (measured as saccharide) in the final mixture is between 1 and 20 µg/ml *e.g.* between 5 and 15 µg/ml, such as aruond 8µg/ml. An optional aluminum salt adjuvant may be added at this stage (*e.g.* to give a final Al³⁺ concentration of between 0.4 and 0.5 mg/ml)

After mixing, the mixed conjugates can be sterile filtered.

### Pharmaceutical compositions

Conjugates prepared by methods of the invention can be combined with pharmaceutically acceptable carriers. Such carriers include any carrier that does not itself induce the production of antibodies harmful to the individual receiving the composition. Suitable carriers are typically large, slowly metabolised macromolecules such as proteins, polysaccharides, polylactic acids, polyglycolic acids, polymeric amino acids, amino acid copolymers, sucrose, trehalose, lactose, and lipid aggregates (such as oil droplets or liposomes). Such carriers are well known to those of ordinary skill in the art. The vaccines may also contain diluents, such as water, saline, glycerol, *etc.* Additionally, auxiliary substances, such as wetting or emulsifying agents, pH buffering substances, and the like, may be present. Sterile pyrogen-free, phosphate-buffered physiologic saline is a typical carrier. A thorough discussion of pharmaceutically acceptable excipients is available in reference 67.

Compositions may include an antimicrobial, particularly if packaged in a multiple dose format.

Compositions may comprise detergent *e.g.* a Tween (polysorbate), such as Tween 80. Detergents are generally present at low levels *e.g.* <0.01%.

Compositions may include sodium salts (*e.g.* sodium chloride) to give tonicity. A concentration of 10±2mg/ml NaCl is typical.

Compositions will generally include a buffer. A phosphate buffer is typical.

Compositions may comprise a sugar alcohol *(e.g.* mannitol) or a disaccharide *(e.g.* sucrose or trehalose) *e.g.* at around 15-30mg/ml (*e.g.* 25 mg/ml), particularly if they are to be lyophilised or if they include material which has been reconstituted from lyophilised material. The pH of a composition for lyophilisation may be adjusted to around 6.1 prior to lyophilisation.

Conjugates may be administered in conjunction with other immunoregulatory agents. In particular, compositions will usually include a vaccine adjuvant. Adjuvants which may be used in compositions of the invention include, but are not limited to:

### A. Mineral-containing compositions

Mineral containing compositions suitable for use as adjuvants in the invention include mineral salts, such as aluminium salts and calcium salts. The invention includes mineral salts such as hydroxides (*e.g.* oxyhydroxides), phosphates (*e.g.* hydroxyphosphates, orthophosphates), sulphates, *etc.* [*e.g.* see chapters 8 & 9 of ref. 68], or mixtures of different mineral compounds, with the compounds taking any suitable form *(e.g.* gel, crystalline, amorphous, *etc.),* and with adsorption being preferred. The mineral containing compositions may also be formulated as a particle of metal salt [69].

Aluminium phosphates are particularly preferred, particularly in compositions which include a *H.influenzae* saccharide antigen, and a typical adjuvant is amorphous aluminium hydroxyphosphate with PO₄/Al molar ratio between 0.84 and 0.92, included at 0.6mg Al³⁺/ml. Adsorption with a low dose of aluminium phosphate may be used *e.g.* between 50 and 100µg Al³⁺ per conjugate per dose. Where there is more than one conjugate in a composition, not all conjugates need to be adsorbed.

### B. Oil Emulsions

Oil emulsion compositions suitable for use as adjuvants in the invention include squalene-water emulsions, such as MF59 [Chapter 10 of ref. 68; see also ref. 70] (5% Squalene, 0.5% Tween 80, and 0.5% Span 85, formulated into submicron particles using a microfluidizer). Complete Freund's adjuvant (CFA) and incomplete Freund's adjuvant (IFA) may also be used.

### C. Saponin formulations [chapter 22 of ref. 68]

Saponin formulations may also be used as adjuvants in the invention. Saponins are a heterologous group of sterol glycosides and triterpenoid glycosides that are found in the bark, leaves, stems, roots and even flowers of a wide range of plant species. Saponin from the bark of the *Quillaia saponaria* Molina tree have been widely studied as adjuvants. Saponin can also be commercially obtained from *Smilax ornata* (sarsaprilla), *Gypsophilla paniculata* (brides veil), and *Saponaria officianalis* (soap root). Saponin adjuvant formulations include purified formulations, such as QS21, as well as lipid formulations, such as ISCOMs. QS21 is marketed as Stimulon™.

Saponin compositions have been purified using HPLC and RP-HPLC. Specific purified fractions using these techniques have been identified, including QS7, QS17, QS18, QS21, QH-A, QH-B and QH-C. Preferably, the saponin is QS21. A method of production of QS21 is disclosed in ref. 71. Saponin formulations may also comprise a sterol, such as cholesterol [72].

Combinations of saponins and cholesterols can be used to form unique particles called immunostimulating complexs (ISCOMs) [chapter 23 of ref. 68]. ISCOMs typically also include a phospholipid such as phosphatidylethanolamine or phosphatidylcholine. Any known saponin can be used in ISCOMs. Preferably, the ISCOM includes one or more of QuilA, QHA & QHC. ISCOMs are further described in refs. 72-74. Optionally, the ISCOMS may be devoid of additional detergent [75].

A review of the development of saponin based adjuvants can be found in refs. 76 & 77.

### D. Virosomes and virus-like particles

Virosomes and virus-like particles (VLPs) can also be used as adjuvants in the invention. These structures generally contain one or more proteins from a virus optionally combined or formulated with a phospholipid. They are generally non-pathogenic, non-replicating and generally do not contain any of the native viral genome. The viral proteins may be recombinantly produced or isolated from whole viruses. These viral proteins suitable for use in virosomes or VLPs include proteins derived from influenza virus (such as HA or NA), Hepatitis B virus (such as core or capsid proteins), Hepatitis E virus, measles virus, Sindbis virus, Rotavirus, Foot-and-Mouth Disease virus, Retrovirus, Norwalk virus, human Papilloma virus, HIV, RNA-phages, Qß-phage (such as coat proteins), GA-phage, fr-phage, AP205 phage, and Ty (such as retrotransposon Ty protein pl). VLPs are discussed further in refs. 78-83. Virosomes are discussed further in, for example, ref. 84

### E. Bacterial or microbial derivatives

Adjuvants suitable for use in the invention include bacterial or microbial derivatives such as non-toxic derivatives of enterobacterial lipopolysaccharide (LPS), Lipid A derivatives, immunostimulatory oligonucleotides and ADP-ribosylating toxins and detoxified derivatives thereof. Non-toxic derivatives of LPS include monophosphoryl lipid A (MPL) and 3-O-deacylated MPL (3dMPL). 3dMPL is a mixture of 3 de-O-acylated monophosphoryl lipid A with 4, 5 or 6 acylated chains. A preferred "small particle" form of 3 De-O-acylated monophosphoryl lipid A is disclosed in ref. 85. Such "small particles" of 3dMPL are small enough to be sterile filtered through a 0.22µm membrane [85]. Other non-toxic LPS derivatives include monophosphoryl lipid A mimics, such as aminoalkyl glucosaminide phosphate derivatives *e.g.* RC-529 [86,87].

Lipid A derivatives include derivatives of lipid A from *Escherichia coli* such as OM-174. OM-174 is described for example in refs. 88 & 89.

Immunostimulatory oligonucleotides suitable for use as adjuvants in the invention include nucleotide sequences containing a CpG motif (a dinucleotide sequence containing an unmethylated cytosine linked by a phosphate bond to a guanosine). Double-stranded RNAs and oligonucleotides containing palindromic or poly(dG) sequences have also been shown to be immunostimulatory.

The CpG's can include nucleotide modifications/analogs such as phosphorothioate modifications and can be double-stranded or single-stranded. References 90, 91 and 92 disclose possible analog substitutions *e.g.* replacement of guanosine with 2'-deoxy-7-deazaguanosine. The adjuvant effect of CpG oligonucleotides is further discussed in refs. 93-98.

The CpG sequence may be directed to TLR9, such as the motif GTCGTT or TTCGTT [99]. The CpG sequence may be specific for inducing a Th1 immune response, such as a CpG-A ODN, or it may be more specific for inducing a B cell response, such a CpG-B ODN. CpG-A and CpG-B ODNs are discussed in refs. 100-102. Preferably, the CpG is a CpG-A ODN.

Preferably, the CpG oligonucleotide is constructed so that the 5' end is accessible for receptor recognition. Optionally, two CpG oligonucleotide sequences may be attached at their 3' ends to form "immunomers". See, for example, refs. 99 & 103-105.

Bacterial ADP-ribosylating toxins and detoxified derivatives thereof may be used as adjuvants in the invention. Preferably, the protein is derived from *E.coli (E. coli* heat labile enterotoxin "LT"), cholera ("CT"), or pertussis ("PT"). The use of detoxified ADP-ribosylating toxins as mucosal adjuvants is described in ref. 106 and as parenteral adjuvants in ref. 107. The toxin or toxoid is preferably in the form of a holotoxin, comprising both A and B subunits. Preferably, the A subunit contains a detoxifying mutation; preferably the B subunit is not mutated. Preferably, the adjuvant is a detoxified LT mutant such as LT-K63, LT-R72, and LT-G192. The use of ADP-ribosylating toxins and detoxified derivaties thereof, particularly LT-K63 and LT-R72, as adjuvants can be found in refs. 108-115. Numerical reference for amino acid substitutions is preferably based on the alignments of the A and B subunits of ADP-ribosylating toxins set forth in ref. 116, specifically incorporated herein by reference in its entirety.

### F. Human immunomodulators

Human immunomodulators suitable for use as adjuvants in the invention include cytokines, such as interleukins *(e.g.* IL-1, IL-2, IL-4, IL-5, IL-6, IL-7, IL-12 [117], *etc.)* [118], interferons *(e.g.* interferon-γ), macrophage colony stimulating factor, and tumor necrosis factor.

### G. Bioadhesives and Mucoadhesives

Bioadhesives and mucoadhesives may also be used as adjuvants in the invention. Suitable bioadhesives include esterified hyaluronic acid microspheres [119] or mucoadhesives such as cross-linked derivatives of poly(acrylic acid), polyvinyl alcohol, polyvinyl pyrollidone, polysaccharides and carboxymethylcellulose. Chitosan and derivatives thereof may also be used as adjuvants in the invention [120].

### H. Microparticles

Microparticles may also be used as adjuvants in the invention. Microparticles (i.e. a particle of ∼100nm to ∼150µm in diameter, more preferably ∼200nm to ∼30µm in diameter, and most preferably ∼500nm to ∼10µm in diameter) formed from materials that are biodegradable and non-toxic (e.g. a poly(α-hydroxy acid), a polyhydroxybutyric acid, a polyorthoester, a polyanhydride, a polycaprolactone, *etc.),* with poly(lactide-co-glycolide) are preferred, optionally treated to have a negatively-charged surface *(e.g.* with SDS) or a positively-charged surface *(e.g.* with a cationic detergent, such as CTAB).

### I. Liposomes (Chapters 13 & 14 of ref. 68)

Examples of liposome formulations suitable for use as adjuvants are described in refs. 121-123.

### J. Polyoxyethylene ether and polyoxyethylene ester formulations

Adjuvants suitable for use in the invention include polyoxyethylene ethers and polyoxyethylene esters [124]. Such formulations further include polyoxyethylene sorbitan ester surfactants in combination with an octoxynol [125] as well as polyoxyethylene alkyl ethers or ester surfactants in combination with at least one additional non-ionic surfactant such as an octoxynol [126]. Preferred polyoxyethylene ethers are selected from the following group: polyoxyethylene-9-lauryl ether (laureth 9), polyoxyethylene-9-steoryl ether, polyoxytheylene-8-steoryl ether, polyoxyethylene-4-lauryl ether, polyoxyethylene-35-lauryl ether, and polyoxyethylene-23-lauryl ether.

### K Polyphosphazene (PCPP)

PCPP formulations are described, for example, in refs. 127 and 128.

### L. Muramyl peptides

Examples of muramyl peptides suitable for use as adjuvants in the invention include N-acetyl-muramyl-L-threonyl-D-isoglutamine (thr-MDP), N-acetyl-normuramyl-L-alanyl-D-isoglutamine (nor-MDP), and N-acetylmuramyl-L-alanyl-D-isoglutaminyl-L-alanine-2-(1'-2'-dipalmitoyl-*sn*-glycero-3-hydroxyphosphoryloxy)-ethylamine MTP-PE).

### M. Imidazoquinolone Compounds.

Examples of imidazoquinolone compounds suitable for use adjuvants in the invention include Imiquamod and its homologues (*e,g*. "Resiquimod 3M"), described further in refs. 129 and 130.

The invention may also comprise combinations of aspects of one or more of the adjuvants identified above. For example, the following adjuvant compositions may be used in the invention: (1) a saponin and an oil-in-water emulsion [131]; (2) a saponin (*e.g*. QS21) + a non-toxic LPS derivative *(e.g.* 3dMPL) [132]; (3) a saponin (*e.g.* QS21) + a non-toxic LPS derivative (*e.g.* 3dMPL) + a cholesterol; (4) a saponin (*e.g.* QS21) + 3dMPL + IL-12 (optionally + a sterol) [133]; (5) combinations of 3dMPL with, for example, QS21 and/or oil-in-water emulsions [134]; (6) SAF, containing 10% squalane, 0.4% Tween 80™, 5% pluronic-block polymer L121, and thr-MDP, either microfluidized into a submicron emulsion or vortexed to generate a larger particle size emulsion. (7) Ribi™ adjuvant system (RAS), (Ribi Immunochem) containing 2% squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™); and (8) one or more mineral salts (such as an aluminum salt) + a non-toxic derivative of LPS (such as 3dMPL).

Other substances that act as immunostimulating agents are disclosed in chapter 7 of ref. 68.

The use of an aluminium hydroxide or aluminium phosphate adjuvant is particularly preferred, and antigens are generally adsorbed to these salts. Calcium phosphate is another preferred adjuvant.

The pH of compositions is preferably between 6 and 8, preferably about 7. Stable pH may be maintained by the use of a buffer. Where a composition comprises an aluminium hydroxide salt, it is preferred to use a histidine buffer [135]. The composition may be sterile and/or pyrogen-free. Compositions may be isotonic with respect to humans.

Compositions may be presented in vials, or they may be presented in ready-filled syringes. The syringes may be supplied with or without needles. A syringe will include a single dose of the composition, whereas a vial may include a single dose or multiple doses. Injectable compositions will usually be liquid solutions or suspensions. Alternatively, they may be presented in solid form (*e.g.* freeze-dried) for solution or suspension in liquid vehicles prior to injection.

Compositions may be packaged in unit dose form or in multiple dose form. For multiple dose forms, vials are preferred to pre-filled syringes. Effective dosage volumes can be routinely established, but a typical human dose of the composition for injection has a volume of 0.5ml.

Where a composition is to be prepared extemporaneously prior to use (*e.g.* where a component is presented in lyophilised form) and is presented as a kit, the kit may comprise two vials, or it may comprise one ready-filled syringe and one vial, with the contents of the syringe being used to reactivate the contents of the vial prior to injection.

Immunogenic compositions used as vaccines comprise an immunologically effective amount of antigen(s), as well as any other components, as needed. By 'immunologically effective amount', it is meant that the administration of that amount to an individual, either in a single dose or as part of a series, is effective for treatment or prevention. This amount varies depending upon the health and physical condition of the individual to be treated, age, the taxonomic group of individual to be treated (*e.g.* non-human primate, primate, *etc.),* the capacity of the individual's immune system to synthesise antibodies, the degree of protection desired, the formulation of the vaccine, the treating doctor's assessment of the medical situation, and other relevant factors. It is expected that the amount will fall in a relatively broad range that can be determined through routine trials, and a typical quantity of each meningococcal conjugate is between 1µg and 20µg per conjugate (measured as saccharide).

Thus the invention provides a method for preparing a pharmaceutical composition, comprising the steps of: (a) preparing a conjugate as described above; (b) mixing the conjugate with one or more pharmaceutically acceptable carriers.

The invention further provides a method for preparing a pharmaceutical product, comprising the steps of: (a) preparing a conjugate as described above; (b) mixing the conjugate with one or more pharmaceutically acceptable carriers; and (c) packaging the conjugate/carrier mixture into a container, such as a vial or a syringe, to give a pharmaceutical product. Insertion into a syringe may be performed in a factory or in a surgery.

Also disclosed herein is a method for prepating a pharmaceutical composition from a saccharide-protein conjugate, comprising the step of admixing the conjugate with a pharmaceutically aacceptable carrier, wherein the conjugate has been prepared by a process conjugation method as described above. The conjugation method and the admixing step can be performed at very different times by different people in different places (*e.g.* in different countries).

Also disclosed herein is a method for packaging a saccharide-protein conjugate into a pharmaceutical product, wherein the conjugate has been prepared by a process conjugation method as described above. The conjugation method and the packacing step can be performed at very different times by different people in different places (*e.g.* in different countries).

### Pharmaceutical uses

Also disclosed herein is a method of treating a patient, comprising preparing a composition as described above, and administering the composition to the patient. The patient may either be at risk from the disease themselves or may be a pregnant woman ('maternal immunisation'). The patient is preferably a human. The human can be of any age *e.g.* <2 years old, from 2-11 years old, from 11-55 years old, >55 years old, *etc.*

Compositions will generally be administered directly to a patient. Direct delivery may be accomplished by parenteral injection (*e.g*. subcutaneously, intraperitoneally, intravenously, intramuscularly, or to the interstitial space of a tissue), or by rectal, oral, vaginal, topical, transdermal, intranasal, ocular, aural, pulmonary or other mucosal administration. Intramuscular administration (*e.g* to the thigh or the upper arm) is preferred. Injection may be via a needle (*e.g*. a hypodermic needle), but needle-free injection may alternatively be used. A typical intramuscular dose is 0.5 ml.

The conjugates prepared by the methods of the invention may be used to elicit systemic and/or mucosal immunity.

Dosage treatment can be a single dose schedule or a multiple dose schedule. Multiple doses may be used in a primary immunisation schedule and/or in a booster immunisation schedule. A primary dose schedule may be followed by a booster dose schedule. Suitable timing between priming doses (*e.g.* between 4-16 weeks), and between priming and boosting, can be routinely determined.

Bacterial infections affect various areas of the body and so compositions may be prepared in various forms. For example, the compositions may be prepared as injectables, either as liquid solutions or suspensions. Solid forms suitable for solution in, or suspension in, liquid vehicles prior to injection can also be prepared (*e.g.* a lyophilised composition). The composition may be prepared for topical administration *e.g.* as an ointment, cream or powder. The composition be prepared for oral administration *e.g.* as a tablet or capsule, or as a syrup (optionally flavoured). The composition may be prepared for pulmonary administration *e.g.* as an inhaler, using a fine powder or a spray. The composition may be prepared as a suppository or pessary. The composition may be prepared for nasal, aural or ocular administration *e.g.* as spray, drops, gel or powder [*e.g.* refs 136 & 137]. Injectable compositions are preferred.

### Further antigenic components of compositions of the invention

The methods of the invention may also comprise the steps of mixing a meningococcal conjugate with one or more of the following further antigens:
- a saccharide antigen from *Haemophilus influenzae B* [*e.g.* chapter 14 of ref. 138].
- a saccharide antigen from *Streptococcus pneumoniae* [*e.g.* 139, 140, 141].
- a purified protien antigen from serogroup B of *Neisseria meningitidis.*
- an outer membrane preparation from serogroup B of *Neisseria meningitidis.*
- an antigen from hepatitis A virus, such as inactivated virus [*e.g.* 142, 143].
- an antigen from hepatitis B virus, such as the surface and/or core antigens [*e.g.* 143, 144].
- a diphtheria antigen, such as a diphtheria toxoid [*e.g*. chapter 13 of ref. 138]
- a tetanus antigen, such as a tetanus toxoid [*e.g.* chapter 27 of ref. 138].
- an antigen from *Bordetella pertussis,* such as pertussis holotoxin (PT) and filamentous haemagglutinin (FHA) from *B.pertussis,* optionally also in combination with pertactin and/or agglutinogens 2 and 3 [*e.g.* refs. 145 & 146; chapter 21 of ref. 138].
- polio antigen(s) [*e.g.* 147,148] such as IPV [chapter 24 of ref. 138].
- measles, mumps and/or rubella antigens [*e.g.* chapters 19, 20 & 26 of ref. 138].
- influenza antigen(s) [*e.g.* chapter 17 of ref. 138], such as the haemagglutinin and/or neuraminidase surface proteins.
- an antigen from *Moraxella catarrhalis* [*e.g.* 149].
- an protein antigen from *Streptococcus agalactiae* (group B streptococcus) [*e.g.* 150, 151].
- a saccharide antigen from *Streptococcus agalactiae* (group B streptococcus).
- an antigen from *Streptococcus pyogenes* (group A streptococcus) [*e.g.* 151, 152, 153].
- an antigen from *Staphylococcus aureus* [*e.g. 154*]*.*

The composition may comprise one or more of these further antigens.

Toxic protein antigens may be detoxified where necessary (*e.g.* detoxification of pertussis toxin by chemical and/or genetic means [146]).

Where a diphtheria antigen is included in the composition it is preferred also to include tetanus antigen and pertussis antigens. Similarly, where a tetanus antigen is included it is preferred also to include diphtheria and pertussis antigens. Similarly, where a pertussis antigen is included it is preferred also to include diphtheria and tetanus antigens. DTP combinations are thus preferred.

Antigens in the composition will typically be present at a concentration of at least 1µg/ml each. In general, the concentration of any given antigen will be sufficient to elicit an immune response against that antigen.

As an alternative to using proteins antigens in the immunogenic compositions of the invention, nucleic acid (preferably DNA *e.g.* in the form of a plasmid) encoding the antigen may be used.

Antigens are preferably adsorbed to an aluminium salt.

Two preferred non-meningococcal antigens for inclusion in compositions are those which protect against *Haemophilus influenzae* type B (Hib) and *Streptococcus pneumoniae.*

### Haemophilus influenzae type B (Hib)

Where the composition includes a *H.influenzae* type B antigen, it will typically be a Hib capsular saccharide antigen. Saccharide antigens from *H.influenzae* b are well known.

Advantageously, the Hib saccharide is covalently conjugated to a carrier protein, in order to enhance its immunogenicity, especially in children. The preparation of polysaccharide conjugates in general, and of the Hib capsular polysaccharide in particular, is well documented. The invention may use any suitable Hib conjugate. Suitable carrier proteins are described above, and preferred carriers for Hib saccharides are CRM₁₉₇ ('HbOC'), tetanus toxoid ('PRP-T') and the outer membrane complex of *N.meningitidis* ('PRP-OMP').

The saccharide moiety of the conjugate may be a polysaccharide (*e.g.* full-length polyribosylribitol phosphate (PRP)), but it is preferred to hydrolyse polysaccharides to form oligosaccharides (*e.g.* MW from ∼1 to ∼5 kDa).

A preferred conjugate comprises a Hib oligosaccharide covalently linked to CRM₁₉₇ via an adipic acid linker [155, 156]. Tetanus toxoid is also a preferred carrier.

Administration of the Hib antigen preferably results in an anti-PRP antibody concentration of ≥0.15µg/ml, and more preferably ≥1µg/ml.

Where a composition includes a Hib saccharide antigen, it is preferred that it does not also include an aluminium hydroxide adjuvant. If the composition includes an aluminium phosphate adjuvant then the Hib antigen may be adsorbed to the adjuvant [157] or it may be non-adsorbed [158]. Prevention of adsorption can be achieved by selecting the correct pH during antigen/adjuvant mixing, an adjuvant with an appropriate point of zero charge, and an appropriate order of mixing for the various different antigens in a composition [159].

Compositions of the invention may comprise more than one Hib antigen. Hib antigens may be lyophilised *e.g.* for reconstitution by meningococcal compositions. Thus a Hib antigen may be packaged separately from meningococcal conjugates, or may be admixed with them.

### Streptococcus pneumoniae

Where the composition includes a *S.pneumoniae* antigen, it will typically be a capsular saccharide antigen which is preferably conjugated to a carrier protein [*e.g.* refs. 139 to 141]. It is preferred to include saccharides from more than one serotype of *S.pneumoniae.* For example, mixtures of polysaccharides from 23 different serotype are widely used, as are conjugate vaccines with polysaccharides from between 5 and 11 different serotypes [161]. For example, PrevNar™ [162] contains antigens from seven serotypes (4, 6B, 9V, 14, 18C, 19F, and 23F) with each saccharide individually conjugated to CRM₁₉₇ by reductive amination, with 2µg of each saccharide per 0.5ml dose (4µg of serotype 6B), and with conjugates adsorbed on an aluminium phosphate adjuvant. Compositions of the invention preferably include at least serotypes 6B, 14, 19F and 23F. Conjugates may be adsorbed onto an aluminium phosphate.

As an alternative to using saccharide antigens from pneumococcus, the composition may include one or more polypeptide antigens. Genome sequences for several strains of pneumococcus are available [163,164] and can be subjected to reverse vaccinology [165-168] to identify suitable polypeptide antigens [169,170]. For example, the composition may include one or more of the following antigens: PhtA, PhtD, PhtB, PhtE, SpsA, LytB, LytC, LytA, Sp125, Sp101, Sp128, Sp130 and Sp133, as defined in reference 171. The composition may include more than one (*e.g.* 2, 3, 4, 5, 6, 7, 8, 9 10, 11, 12, 13 or 14) of these antigens.

In some embodiments, the composition may include both saccharide and polypeptide antigens from pneumococcus. These may be used in simple admixture, or the pneumococcal saccharide antigen may be conjugated to a pneumococcal protein. Suitable carrier proteins for such embodiments include the antigens listed in the previous paragraph [171].

Pneumococcal antigens may be lyophilised e.g. together with Hib antigen.

### General

The term "comprising" encompasses "including" as well as "consisting" *e.g.* a composition "comprising" X may consist exclusively of X or may include something additional *e.g.* X + Y.

The term "about" in relation to a numerical value x means, for example, *x*±10%.

The word "substantially" does not exclude "completely" *e.g.* a composition which is "substantially free" from Y may be completely free from Y. Where necessary, the word "substantially" may be omitted from the definition of the invention.

### MODES FOR CARRYING OUT THE INVENTION

The skilled person will be able to implement the invention without specific guidance, following for instance the teaching of references 8, 9 and 12. Two simple examples are provided below, but it will be understood that these examples are illustrative only.

Meningococci from serogroups A, C, W135 and Y are separately grown in media as defined above. Capsular polysaccharides are purified from the bacteria. Each purified saccharide is subjected to optional depolymerisation. Each saccharide is activated, and covalently linked to a carrier protein (*e.g*. to diphtheria toxoid, prepared as described in ref. 8; or to CRM₁₉₇). The four separate conjugates are then combined at a mass ratio (A:C:W135:Y) of either 2:1:1:1 or 1:1:1:1. The mixed conjugates are then used for immunisation.

In a second preparation, the conjugates of serogroups C, W135 and Y are mixed at a 1:1:1 mass ratio. The serogroup A conjugate is lyophilised, and can be reconstituted by the mixed C/W135/Y conjugates.

### REFERENCES

[1] Armand et al. (1982) J. Biol. Stand. 10:335-339.
[2] Cadoz et al. (1985) Vaccine 3:340-342.
[3] MMWR (1997) 46(RR-5) 1-10.
[4] Baklaic et al. (1983) Infect. Immun. 42:599-604.
[5] Jones (2001) Curr Opin Investig Drugs 2:47-49.
[6] Costantino et al. (1992) Vaccine 10:691-8.
[7] Lieberman et al. (1996) JAMA 275:1499-503.
[8] WO02/058737.
[9] WO03/007985.
[10] Rennels et al. (2002) Pediatr Infect Dis J21:978-979.
[11] Campbell et al. (2002) J Infect Dis 186:1848-1851.
[12] WO2004/033623.
[13] WO98/32873.
[14] US patent 4,753,796.
[15] Frash (1990) p.123-145 of Advances in Biotechnological Processes vol. 13 (eds. Mizrahi & Van Wezel)
[16] European patent 0072513.
[17] Inzana (1987) Infect. Immun. 55:1573-1579.
[18] Ramsay et al. (2001) Lancet 357(9251):195-196.
[19] Lindberg (1999) Vaccine 17 Suppl 2:S28-36.
[20] Buttery & Moxon (2000) JR Coll Physicians Lond 34:163-168.
[21] Ahmad & Chapnick (1999) Infect Dis Clin North Am 13:113-33, vii.
[22] Goldblatt (1998) J. Med. Microbiol. 47:563-567.
[23] European patent 0477508.
[24] US patent 5,306,492.
[25] WO98/42721.
[26] Dick et al. in Conjugate Vaccines (eds. Cruse et al.) Karger, Basel, 1989, 10:48-114.
[27] Hermanson Bioconjugate Techniques, Academic Press, San Diego (1996) ISBN: 0123423368.
[28] Anonymous (Jan 2002) *Research Disclosure,* 453077.
[29] Anderson (1983) Infect Immun 39(1):233-238.
[30] Anderson et al. (1985) J Clin Invest 76(1):52-59.
[31] EP-A-0372501.
[32] EP-A-0378881.
[33] EP-A-0427347.
[34] WO93/17712
[35] WO94/03208.
[36] WO98/58668.
[37] EP-A-0471177.
[38] WO91/01146
[39] Falugi et al. (2001) Eur J Immunol 31:3816-3824*.*
[40] EP-A-0594610.
[41] WO00/56360.
[42] WO02/091998.
[43] WO01/72337
[44] WO00/61761.
[45] WO99/42130
[46] WO96/40242
[47] Lees et al. (1996) Vaccine 14:190-198.
[48] WO95/08348.
[49] US patent 4,882,317
[50] US patent 4,695,624
[51] Porro et al. (1985) Mol Immunol 22:907-919.
[52] EP-A-0208375
[53] WO00/10599
[54] Gever et al. Med. Microbiol. Immunol, 165 : 171-288 (1979).
[55] US patent 4,057,685.
[56] US patents 4,673,574; 4,761,283; 4,808,700.
[57] US patent 4,459,286.
[58] US patent 4,965,338
[59] US patent 4,663,160.
[60] US patent 4,761,283
[61] US patent 4,356,170
[62] Lei et al. (2000) Dev Biol (Basel) 103:259-264.
[63] WO00/38711; US patent 6,146,902.
[64] Lamb et al. (2000) Dev Biol (Basel) 103:251-258.
[65] Lamb et al. (2000) Journal of Chromatography A 894:311-318.
[66] D'Ambra et al. (2000) Dev Biol (Basel) 103:241-242.
[67] Gennaro (2000) Remington: The Science and Practice of Pharmacy. 20th edition, ISBN: 0683306472.
[68] Vaccine Design... (1995) eds. Powell & Newman. ISBN: 030644867X. Plenum.
[69] WO00/23105.
[70] WO90/14837.
[71] US patent 5,057,540.
[72] WO96/33739.
[73] EP-A-0109942.
[74] WO96/11711.
[75] WO00/07621.
[76] Barr et al. (1998) Advanced Drug Delivery Reviews 32:247-271.
[77] Sjolanderet et al. (1998) Advanced Drug Delivery Reviews 32:321-338.
[78] Niikura et al. (2002) Virology 293:273-280.
[79] Lenz et al. (2001) J Immunol 166:5346-5355.
[80] Pinto et al. (2003) J Infect Dis 188:327-338.
[81] Gerber et al. (2001) Virol 75:4752-4760.
[82] WO03/024480
[83] WO03/024481
[84] Gluck et al. (2002) Vaccine 20:B10-B16.
[85] EP-A-0689454.
[86] Johnson et al. (1999) Bioorg Med Chem Lett 9:2273-2278.
[87] Evans et al. (2003) Expert Rev Vaccines 2:219-229*.*
[88] Meraldi et al. (2003) Vaccine 21:2485-2491.
[89] Pajak et al. (2003) Vaccine 21:836-842.
[90] Kandimalla et al. (2003) Nucleic Acids Research 31:2393-2400.
[91] WO02/26757.
[92] WO99/62923.
[93] Krieg (2003) Nature Medicine 9:831-835*.*
[94] McCluskie et al. (2002) FEMS Immunology and Medical Microbiology 32:179-185.
[95] WO98/40100.
[96] US patent 6,207,646.
[97] US patent 6,239,116.
[98] US patent 6,429,199.
[99] Kandimalla et al. (2003) Biochemical Society Transactions 31 (part 3):654-658.
[100] Blatkwell et al. (2003) J Immunol 170:4061-4068.
[101] Krieg (2002) Trends Immunol 23:64-65.
[102] WO01/95935.
[103] Kandimalla et al. (2003) BBRC 306:948-953.
[104] Bhagat et al. (2003) BBRC 300:853-861.
[105] WO03/035836.
[106] WO95/17211.
[107] WO98/42375.
[108] Beignon et al. (2002) Infect Immun 70:3012-3019.
[109] Pizza et al. (2001) Vaccine 19:2534-2541.
[110] Pizza et al. (2000) Int J Med Microbiol 290:455-461.
[111] Scharton-Kersten et al. (2000) Infect Immun 68:5306-5313.
[112] Ryan et al. (1999) Infect Immun 67:6270-6280.
[113] Partidos et al. (1999) Immunol Lett 67:209-216.
[114] Peppoloni et al. (2003) Expert Rev Vaccines 2:285-293.
[115] Pine et al. (2002) J Control Release 85:263-270.
[116] Domenighini et al. (1995) Mol Microbiol 15:1165-1167*.*
[117] WO99/40936.
[118] WO99/44636.
[119] Singh et al] (2001) J Cont Release 70:267-276.
[120] WO99/27960.
[121] US patent 6,090,406
[122] US patent 5,916,588
[123] EP-A-0626169.
[124] WO99/52549.
[125] WO01/21207.
[126] WO01/21152.
[127] Andrianov et al. (1998) Biomaterials 19:109-115*.*
[128] Payne et al. (1998) Adv Drug Delivery Review 31:185-196.
[129] Stanley (2002) Clin Exp Dermatol 27:571-577.
[130] Jones (2003) Curr Opin Investig Drugs 4:214-218.
[131] WO99/11241.
[132] WO94/00153.
[133] WO98/57659.
[134] European patent applications 0835318, 0735898 and 0761231.
[135] WO03/009869.
[136] Almeida & Alpar (1996) J. Drug Targeting 3:455-467.
[137] Agarwal & Mishra (1999) Indian J Exp Biol 37:6-16.
[138] Vaccines (2004) eds. Plotkin & Orenstein. ISBN 0-7216-9688-0.
[139] Watson (2000) Pediatr Infect Dis J 19:331-332.
[140] Rubin (2000) Pediatr Clin North Am 47:269-285, v.
[141] Jedrzejas (2001) Microbiol Mol Biol Rev 65:187-207.
[142] Bell (2000) Pediatr Infect Dis J 19:1187-1188.
[143] Iwarson (1995) APMIS 103:321-326.
[144] Gerlich et al. (1990) Vaccine 8 Suppl:S63-68 & 79-80.
[145] Gustafsson et al. (1996) N. Engl. J. Med. 334:349-355.
[146] Rappuoli et al. (1991) TIBTECH9:232-238*.*
[147] Sutter et al. (2000) Pediatr Clin North Am 47:287-308.
[148] Zimmerman & Spann (1999) Am Fam Physician 59:113-118, 125-126.
[149] McMichael (2000) Vaccine 19 Suppl 1:S101-107.
[150] Schuchat (1999) Lancet 353(9146):51-6.
[151] WO02/34771.
[152] Dale (1999) Infect Dis Clin North Am 13:227-43, viii.
[153] Ferretti et al. (2001) PNAS USA 98: 4658-4663.
[154] Kuroda et al. (2001) Lancet 357(9264):1225-1240; see also pages 1218-1219.
[155] Kanra et al. (1999) The Turkish Journal of Paediatrics 42:421-427.
[156] Ravenscroft et al. (2000) Dev Biol (Basel) 103: 35-47.
[157] WO97/00697.
[158] WO02/00249.
[159] WO96/37222; US patent 6,333,036.
[160] Rubin (2000) Pediatr Clin NorthAm 47:269-285, v.
[161] Zielen et al. (2000) Infect. Immun. 68:1435-1440.
[162] Darkes & Plosker (2002) Paediatr Drugs 4:609-630.
[163] Tettelin et al. (2001) Science 293:498-506.
[164] Hoskins et al (2001) J Bacteriol 183:5709-5717.
[165] Rappuoli (2000) Curr Opin Microbiol 3:445-450
[166] Rappuol (2001) Vaccine 19:2688-2691.
[167] Masignani et al. (2002) Expert Opin Biol Ther 2:895-905.
[168] Mora et al. (2003) Drug Discov Today 8:459-464.
[169] Wizemann et al. (2001) Infect Immun 69:1593-1598.
[170] Rigden et al. (2003) Crit Rev Biochem Mol Biol 38:143-168.
[171] WO02/22167.
[172] WO98/54296.

## Claims

1. A method for preparing a protein-saccharide conjugate, comprising the steps of:
(a) preparing an aqueous growth medium comprising either
a. (i). 2.5 g/l sodium phosphate, dibasic; (ii) between 5 and 30 g/l soy peptone; (iii) 5 g/l monosodium glutamate; (iv) 0.103 g/l potassium chloride; (v) 0.732 g/l magnesium sulfate; (vi) 11.250 g/l glucose; and, optionally, (vii) 0.016 g/l L-cysteine, or
b. (i) 10 g/l glucose; (ii) between 5 and 30 g/l soy peptone; (iii) 5.80 g/l sodium chloride; (iv) 1 g/l potassium sulfate; (v) 4 g/l potassium phosphate, dibasic; (vi) 0.19 g/l magnesium chloride; (vii) 0.021 g/l calcium chloride; (viii) 0.002 g/l ferrous sulfate; and a mixture of amino acids comprising 5 g/l L-glutamic acid, 0.3 g/l L-arginine, 0.5 g/l L-serine and 0.23 g/l L-cysteine, but not including ammonium chloride;
(b) inoculating the medium with a *Neisseria meningitidis* bacterium;
(c) incubating the medium to allow growth of the bacterium;
(d) preparing capsular saccharide from the bacterium; and
(e) conjugating the capsular saccharide to a carrier protein, to give the a protein-saccharide conjugate;
wherein the concentrations of components of the medium may vary by ±10%.

2. The method of claim (i). 2.5 g/l sodium phosphate, dibasic; (ii) between 5 and 30 g/l soy peptone; (iii) 5 g/l monosodium glutamate; (iv) 0.103 g/l potassium chloride; (v) 0.732 g/l magnesium sulfate; (vi) 11.250 g/l glucose; and, optionally, (vii) 0.016 g/l L-cysteine wherein the medium has pH 6.8±0.2.

3. The method of any preceding claim, wherein the medium includes a foam control agent,

4. The method of any preceding claim, wherein steps (b) and (c) are repeated more than once, with inoculation into fresh medium in each repeat.

5. The method of any preceding claim, wherein step (c) takes place at 30-40°C.

6. The method of any preceding claim, wherein step (c) comprises fed-batch culture.

7. The method of any preceding claim, wherein step (d) comprises: CTAB addition, centrifugation, and collection of supernatant.

8. The method of any preceding claim, wherein step (e) comprises conjugation of the saccharide to a diphtheria toxoid carrier protein.

9. The method of any preceding claim, wherein step (e) comprises: reacting the saccharide with adipic acid dihydrazide; addition of sodium cyanoborohydride; and addition of the carrier protein.

10. The method of any preceding claim, wherein, between steps (d) and (e), the saccharide is treated with hydrogen peroxide to reduce its chain length.

11. The method of any one of claims 1 to 10; wherein the *Neisseria meningitidis* is serogroup A.

12. The method of any one of claims 1 to 10, wherein the *Neisseria meningitidis* is serogroup C.

13. the method of any one of claims 1 to 10, wherein the *Neisseria meningitidis* is serogroup W135.

14. The method of any one of claims 1 to 10, wherein the *Neisseria meningitidis* is serogroup Y.

15. A method for preparing a mixture of conjugates of the capsular saccharides of serogroups A, C, W135 and Y, comprising the steps of: preparing a conjugate by the method of claim 11; preparing a conjugate by the method of claim 12; preparing a conjugate by the method of claim 13. preparing a conjugate by the method of claim 14; and mixing these four conjugates.

16. The method of claim 15, wherein the four conjugates are mixed with a phosphate buffered saline solution.

17. The method of claim 15, claim 16, wherein the four conjugates are mixed with an aluminium hydroxide adjuvant.

18. The method of claim 15 or claim 16, wherein the four conjugates are mixed with an aluminium phosphate adjuvant.

19. A method for preparing a pharmaceutical composition comprising the steps of: (a) preparing a conjugate by the method of any one of claims 1 to 14, or a combination of conjugates by the method of any one of claims 15 to 18; and (b) mixing the conjugate(s) with one or more pharmaceutically acceptable carriers.

20. The method of claim 19, wherein the pharmaceutical composition is packaged for injection.

21. The method of claim 19 or claim 20, further comprising the step of (c) putting the pharmaceutical composition into a syringe.

22. The method of any preceding claim, further comprising the step of mixing a meningococcal conjugate with (a) a *Haemophilus influenzae* type B capsular saccharide conjugate, and/or (b) a *Streptococcus pneumoniae* capsular saccharide conjugate.

## Patentansprüche

1. Verfahren zur Herstellung eines Protein-Saccharid-Konjugats, welches die folgenden Schritte umfasst:
(a) Herstellung eines wässrigen Wachstumsmediums, welches entweder
a.
(i) 2,5 g/l Natriumphosphat, dibasisch; (ii) zwischen 5 und 30 g/l Sojapepton;
(iii) 5 g/l Mononatriumglutamat;
(iv) 0,103 g/l Kaliumchlorid; (v) 0,732 g/l Magnesiumsulfat; (vi) 11,250 g/l Glukose und gegebenenfalls (vii) 0,016 g/l L-Cystein oder
b. (i) 10 g/l Glukose; (ii) zwischen 5 und 30 g/l Sojapepton; (iii) 5,80 g/l Natriumchlorid; (iv) 1 g/l Kaliumsulfat; (v) 4 g/l Kaliumphosphat, dibasisch; (vi) 0,19 g/l Magnesiumchlorid; (vii) 0,021 g/l Kalziumchlorid; (viii) 0, 002 g/l Eisen (II)-Sulfat und eine Aminosäuremischung, welche 5 g/l L-Glutaminsäure, 0,3 g/l L-Arginin, 0,5 g/l L-Serin und 0,23 g/l L-Cystein umfasst,
umfasst, aber kein Ammoniumchlorid umfasst;
(b) Inokulieren des Mediums mit einem *Neisseria meningitidis*-Bakterium;
(c) Inkubieren des Mediums, um das Bakterium wachsen zu lassen;
(d) Isolieren des Kapselsaccharids aus dem Bakterium und
(e) Konjugieren des Kapselsaccharids mit einem Trägerprotein unter Bildung des Protein-Saccharid-Konjugats;
wobei die Konzentrationen der Komponenten des Mediums um ±10% schwanken können.

2. Verfahren nach Anspruch 1, wobei das Medium einen pH-Wert von 6,8±0,2 aufweist.

3. Verfahren nach einem der vorhergehenden Ansprüche, wobei das Medium ein Schaumbekämpfungsmittel einschließt.

4. Verfahren nach einem der vorhergehenden Ansprüche, wobei die Schritte (b) und (c) mehr als einmal wiederholt werden, wobei bei jeder Wiederholung in frisches Medium inokuliert wird.

5. Verfahren nach einem der vorhergehenden Ansprüche, wobei Schritt (c) bei 30-40°C stattfindet.

6. Verfahren nach einem der vorhergehenden Ansprüche, wobei Schritt (c) eine Fed-Batch-Kultur umfasst.

7. Verfahren nach einem der vorhergehenden Ansprüche, wobei Schritt (d) Folgendes umfasst: Zugabe von CTAB, Zentrifugieren und Abnehmen des Überstands.

8. Verfahren nach einem der vorhergehenden Ansprüche, wobei Schritt (e) die Konjugation des Saccharids mit einem Diphtherietoxoid-Trägerprotein umfasst.

9. Verfahren nach einem der vorhergehenden Ansprüche, wobei Schritt (e) Folgendes umfasst: Umsetzung des Saccharids mit Adipinsäuredihydrazid, Zugabe von Natriumcyanoborhydrid und Zugabe des Trägerprote-ins.

10. Verfahren nach einem der vorhergehenden Ansprüche, wobei zwischen den Schritten (d) und (e) das Saccharid mit Wasserstoffperoxid behandelt wird, um seine Kettenlänge zu reduzieren.

11. Verfahren nach einem der Ansprüche 1 bis 10, wobei das *Neisseria meningitidis* Serogruppe A ist.

12. Verfahren nach einem der Ansprüche 1 bis 10, wobei das *Neisseria meningitidis* Serogruppe C ist.

13. Verfahren nach einem der Ansprüche 1 bis 10, wobei das *Neisseria meningitidis* Serogruppe W135 ist.

14. Verfahren nach einem der Ansprüche 1 bis 10, wobei das *Neisseria meningitidis* Serogruppe Y ist.

15. Verfahren zur Herstellung einer Mischung von Konjugaten der Kapselsaccharide der Serogruppen A, C, W135 und Y, welches folgende Schritte umfasst: Herstellung eines Konjugats nach dem Verfahren von Anspruch 11, Herstellung eines Konjugats nach dem Verfahren nach Anspruch 12, Herstellung eines Konjugats nach dem Verfahren von Anspruch 13, Herstellung eines Konjugats nach dem Verfahren von Anspruch 14, und Mischen dieser vier Konjugate.

16. Verfahren nach Anspruch 15, wobei die vier Konjugate mit einer phosphatgepufferten Kochsalz-lösung gemischt werden.

17. Verfahren nach Anspruch 15 oder 16, wobei die vier Konjugate mit einem Aluminiumhydroxidadjuvans gemischt werden.

18. Verfahren nach Anspruch 15 oder Anspruch 16, wobei die vier Konjugate mit einem Aluminiumphosphat-adjuvans gemischt werden.

19. Verfahren zur Herstellung einer pharmazeutischen Zusammensetzung, welches die folgenden Schritte umfasst: (a) Herstellung eines Konjugats durch das Verfahren nach einem der Ansprüche 1 bis 14 oder einer Kombination von Konjugaten nach dem Verfahren nach einem der Ansprüche 15 bis 18 und (b) Mischen des Konjugats/der Konjugate mit einem oder mehreren pharmazeutisch unbedenklichen Trägern.

20. Verfahren nach Anspruch 19, wobei die pharmazeutische Zusammensetzung für die Injektion abgepackt ist.

21. Verfahren nach Anspruch 19 oder 20, welches weiterhin den Schritt (c) des Abfüllens der pharmazeutischen Zusammensetzung in eine Spritze umfasst.

22. Verfahren nach einem der vorhergehenden Ansprüche, welches weiterhin den Schritt des Mischens eines Meningokokkenkonjugats mit (a) einem *Haemophilus influenzae*-Typ-B-Kapselsaccharidkonjugat und/oder (b) einem *Streptococcus pneumoniae-*Kapselsaccharidkonjugat umfasst.

## Revendications

1. Procédé pour préparer un conjugué protéine-saccharide, comprenant les étapes de :
(a) préparation d'un milieu de croissance aqueux comprenant soit
a. (i) 2,5 g/l de phosphate de sodium dibasique ; (ii) entre 5 et 30 g/l de peptone de soja ; (iii) 5 g/l de glutamate monosodique ; (iv) 0,103 g/l de chlorure de potassium ; (v) 0,732 g/l de sulfate de magnésium ; (vi) 11,250 g/l de glucose ; et, facultativement, (vii) 0,016 g/l de L-cystéine, ou
b. (i) 10 g/l de glucose ; (ii) entre 5 et 30 g/l de peptone de soja ; (iii) 5,80 g/l de chlorure de sodium ; (iv) 1 g/l de sulfate de potassium ; (v) 4 g/l de phosphate de potassium dibasique ; (vi) 0,19 g/l de chlorure de magnésium ; (vii) 0,021 g/l de chlorure de calcium ; (viii) 0,002 g/l de sulfate ferreux ; et un mélange d'acides aminés comprenant 5 g/l d'acide L-glutamique, 0,3 g/l de L-arginine, 0,5 g/l de L-sérine et 0,23 g/l de L-cystéine, mais ne comprenant pas de chlorure d'ammonium ;
(b) inoculation du milieu avec une bactérie *Neisseria meningitidis ;*
(c) incubation du milieu pour permettre la croissance de la bactérie ;
(d) préparation de saccharide capsulaire à partir de la bactérie ; et
(e) conjugaison du saccharide capsulaire à une protéine porteuse, pour obtenir le conjugué protéine-saccharide ;
les concentrations des composants du milieu pouvant varier de ±10 %.

2. Procédé de la revendication 1, dans lequel le milieu a un pH de 6,8±0,2.

3. Procédé de l'une quelconque des revendications précédentes, le milieu comprenant un agent antimousse.

4. Procédé de l'une quelconque des revendications précédentes, dans lequel les étapes (b) et (c) sont répétées plus d'une fois, avec inoculation dans du milieu frais dans chaque répétition.

5. Procédé de l'une quelconque des revendications précédentes, dans lequel l'étape (c) est conduite à 30 à 40 °C.

6. Procédé de l'une quelconque des revendications précédentes, dans lequel l'étape (c) comprend une culture semi-discontinue.

7. Procédé de l'une quelconque des revendications précédentes, dans lequel l'étape (d) comprend : l'ajout de CTAB, la centrifugation, et la collecte de surnageant.

8. Procédé de l'une quelconque des revendications précédentes, dans lequel l'étape (e) comprend la conjugaison du saccharide à une protéine porteuse d'anatoxine diphtérique.

9. Procédé de l'une quelconque des revendications précédentes, dans lequel l'étape (e) comprend : la réaction du saccharide avec le dihydrazide d'acide adipique ; l'ajout de cyanoborohydrure de sodium ; et l'ajout de la protéine porteuse.

10. Procédé de l'une quelconque des revendications précédentes, dans lequel, entre les étapes (d) et (e), le saccharide est traité avec du peroxyde d'hydrogène pour réduire sa longueur de chaîne.

11. Procédé de l'une quelconque des revendications 1 à 10, dans lequel *Neisseria meningitidis* est du sérogroupe A.

12. Procédé de l'une quelconque des revendications 1 à 10 dans lequel *Neisseria meningitidis* est du sérogroupe C.

13. Procédé de l'une quelconque des revendications 1 à 10 dans lequel *Neisseria meningitidis* est du sérogroupe W135.

14. Procédé de l'une quelconque des revendications 1 à 10 dans lequel *Neisseria meningitidis* est du sérogroupe Y.

15. Procédé pour préparer un mélange de conjugués des saccharides capsulaires des sérogroupes A, C, W135 et Y, comprenant les étapes de : préparation d'un conjugué par le procédé de la revendication 11, préparation d'un conjugué par le procédé de la revendication 12 ; préparation d'un conjugué par le procédé de la revendication 13 ; préparation d'un conjugué par le procédé de la revendication 14 ; et mélange de ces quatre conjugués.

16. Procédé de la revendication 15, dans lequel les quatre conjugués sont mélangés avec un soluté tamponné par les phosphates.

17. Procédé de la revendication 15 ou la revendication 16, dans lequel les quatre conjugués sont mélangés avec un adjuvant d'hydroxyde d'aluminium.

18. Procédé de la revendication 15 ou la revendication 16, dans lequel les quatre conjugués sont mélangés avec un adjuvant de phosphate d'aluminium.

19. Procédé de préparation d'une composition pharmaceutique, comprenant les étapes de : (a) préparation d'un conjugué par le procédé de l'une quelconque des revendications 1 à 14, ou une combinaison de conjugués par le procédé de l'une quelconque des revendications 15 à 18 ; et (b) le mélange du/des conjugué(s) avec un ou plusieurs véhicules pharmaceutiquement acceptables.

20. Procédé de la revendication 19, dans lequel la composition pharmaceutique est conditionnée pour injection.

21. Procédé de la revendication 19 ou la revendication 20, comprenant en outre l'étape de (c) placement de la composition pharmaceutique dans une seringue.

22. Procédé de l'une quelconque des revendications précédentes, comprenant en outre l'étape de mélange d'un conjugué méningococcique avec (a) un conjugué de saccharide capsulaire de *Haemophilus influenzae* de type B, et/ou (b) un conjugué de saccharide capsulaire de *Streptococcus pneumoniae.*
